# EUROPÄISCHE PATENTSCHRIFT

(11) **EP 1 300 410 B1**
(45) Veröffentlichungstag und Bekanntmachung des Hinweises auf die Patenterteilung: **28.12.2005**
(21) Anmeldenummer: 02021337.7
(22) Anmeldetag: 23.09.2002
(51) Int. Cl.: C07F 11/00, C07F 15/00

(54) **Neue Übergangsmetall-Komplexe mit Diamino-Carbenliganden und deren Einsatz in Übergangsmetallkatalysierten Reaktionen**
Transition metal complexes comprising a diaminocarben ligand and their use in transition metal catalysed reactions
Complexes des métaux de transition comprenant un ligand de type diaminocarben et leur utilisation dans des réactions catalysées par des métaux de transition

(30) Priorität: 02.10.2001 DE 10148722
(43) Veröffentlichungstag der Anmeldung: 09.04.2003
(73) Patentinhaber: LANXESS Deutschland GmbH, 51369 Leverkusen (DE)
(72) Erfinder: Herrmann, Wolfgang A., Prof. Dr. Dr., 85354 Freising (DE); Denk, Karin, 80803 München (DE)

(56) Entgegenhaltungen:
- EP-A- 0 721 953
- ROMEROSA ET AL.: INORG. CHEM., Bd. 36, 1997, Seiten 3784-3786, XP002225825

## Beschreibung

Die Erfindung betrifft neue Übergangsmetallkomplexe, die mindestens einen Diamino-Carbenliganden besitzen, Verfahren zur Herstellung dieser Übergangsmetallkomplexe sowie ihre Verwendung als Katalysatoren in organischen Reaktionen.

Übergangsmetall-Carbenkomplexe sind seit langem bekannt und stellen wichtige Reagenzien im Bereich der organischen Synthesechemie dar. Übergangsmetall-Carbenkomplexe werden u.a. als Katalysatoren in organischen Reaktionen eingesetzt.

In Herrmann et al., Synth. Meth. Organomet. Inorg. Chem. 9, 2000, 84-112 werden N-heterocyclische Übergangsmetall-Carbenkomplexe beschrieben, wobei beispielsweise Palladium(0)-Carbenkomplexe als Katalysatoren für Heck-Reaktionen eingesetzt werden und Ru(II)-Carbenkomplexe vielversprechende Katalysatoren für Reaktionen darstellen, die C-C- oder C-H-Knüpfungen beinhalten.

In WO 00/15339 A1 werden Carbenkomplexe des Rutheniums und des Osmiums beschrieben, die als Katalysatoren in der Olefin-Metathese eingesetzt werden können.

Trotz schon bekannter Übergangsmetall-Carbenkomplexe, die sich als Katalysatoren in organischen Synthesereaktionen eigenen, bestand Bedarf an neuen Übergangsmetall-Carbenkomplexen, die sich insbesondere als Katalysatoren in organischen Synthesereaktionen einsetzen lassen, wobei sie im Hinblick auf großtechnische Synthesen neben hohen Aktivitäten auch hohe Katalysatorstandzeiten aufweisen sollten.

Überraschenderweise wurden nun Verbindungen der allgemeinen Strukturformel (I) gefunden, 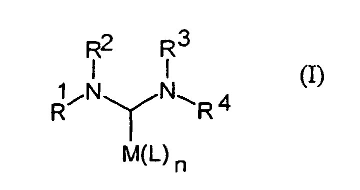 worin
- M: für ein Übergangsmetall der 6. bis 8. Nebengruppe des Periodensystems steht,
- die Reste R¹, R², R³ und R⁴: gleich oder verschieden sind und für Wasserstoff oder/und für Kohlenwasserstoffgruppen stehen, wobei es sich bei den Kohlenwasserstoffgruppen um gleiche oder verschiedene, geradkettige, verzweigte oder cyclische Reste aus der Gruppe Alkylreste mit 1 bis 50 Kohlenstoffatomen, Alkenylreste mit 2 bis 50 Kohlenstoffatomen, Alkinylreste mit 2 bis 50 Kohlenstoffatomen und Arylreste mit 6 bis 30 Kohlenstoffatomen, in denen gegebenenfalls mindestens ein Wasserstoffatom durch eine funktio nelle Gruppe ersetzt ist, handelt und/oder
- R¹ und R² und/oder R³ und R⁴: Teil eines cyclischen Systems sind, welches gleich oder verschieden ist, wobei das cyclische System aus einem Kohlenstoffgerüst mit 2 bis 20 Kohlenstoffatomen und einem Stickstoffatom in Formel (I) besteht, wobei die Kohlenstoffatome von R¹ und R² und/oder R³ und R⁴ in Formel (I) nicht mit eingerechnet werden und wobei gegebenenfalls mindestens ein Wasserstoffatom im Cyclus durch eine funktionelle Gruppe ersetzt ist und/oder gegebenenfalls mindestens ein Kohlenstoffatom des Cyclus durch ein Heteroatom aus der Reihe S, P, O oder N ersetzt ist, und/oder
- R¹ und/oder R² und/oder R³ und/oder R⁴: über eine Brücke von 1 bis 20 Kohlenstoffatomen, wobei die Kohlenstoffatome von R¹, R² R³, und R⁴ nicht mit eingerechnet werden, mit einem der Liganden L verknüpft sind, und
- L: für gleiche oder unabhängig voneinander verschiedene neutrale Zweielektronen-Donoren stehen und/oder Teile eines cyclischen Systems sind und/oder für anionische Liganden stehen, und
- n: für eine ganze Zahl steht, vorzugsweise steht n für 0 bis 6, besonders bevorzugt für 2 bis 4.

Die erfindungsgemäßen Verbindungen weisen als Strukturmerkmal mindestens einen Diamiocarben-Liganden des Typs R¹R²N-C-NR³R⁴ auf und besitzen überraschend hohe Katalysatorstandzeiten. Dieser Vorteil kommt besonders bei Reaktionen, die im Temperaturbereich oberhalb 100°C ablaufen, zum Tragen. Weiterhin weisen die erfindungsgemäßen Verbindungen außerordentliche thermische Stabilitäten und hohe Katalysatoraktivität auf.

Steht L in den erfindungsgemäßen Verbindungen der allgemeinen Formel (I) für gleiche oder unabhängig voneinander verschiedene neutrale Zweielektronen-Donoren, so handelt es sich dabei vorzugsweise um Liganden aus der Reihe Amine, Imine, Phosphane, Phosphite, Stibine, Arsine, CO, Carbonylverbindungen, Nitrile, Alkohole, Thiole, Ether, Thioether und Pyridine.

Weiterhin bevorzugt steht L für gleiche oder unabhängig voneinander verschiedene neutrale Zweielektronen-Donoren Liganden aus der Gruppe der N-heterocyclischen Carbene der allgemeinen Formeln (A - E) 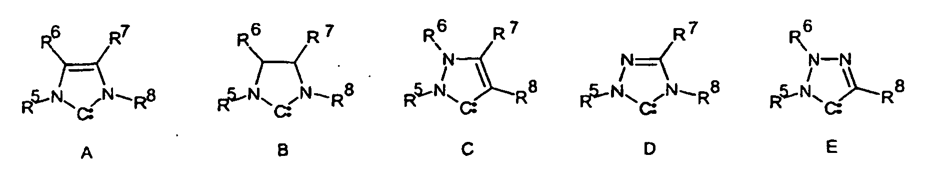 worin
- die Reste R⁵, R⁶, R⁷ und R⁸: gleich oder verschieden sind und für Wasserstoff oder/und für Kohlenwasserstoffgruppen stehen, wobei es sich bei den Kohlenwasserstoffgruppen um gleiche oder verschiedene, geradkettige, verzweigte oder cyclische Reste aus der Gruppe Alkylreste mit 1 bis 50 Kohlenstoffatomen, Alkenylreste mit 2 bis 50 Kohlenstoffatomen, Alkinylreste mit 2 bis 50 Kohlenstoffatomen und Arylreste mit 6 bis 30 Kohlenstoffatomen, in denen gegebenenfalls mindestens ein Wasserstoffatom durch eine funktionelle Gruppe ersetzt ist, handelt und/oder
- die Reste R⁶ und R⁷: für gleiche oder unabhängig voneinander verschiedene Halogen-, Nitro-, Nitroso-, Alkoxy-, Aryloxy-, Amido-, Carboxyl-, Carbonyl-, Thio- oder/und Sulfonylgruppen stehen.

Weiterhin bevorzugt steht L für gleiche oder unabhängig voneinander verschiedene neutrale Zweielektronen-Donor-Liganden aus der Gruppe der Diaminocarben-Liganden der allgemeinen Formel (II), 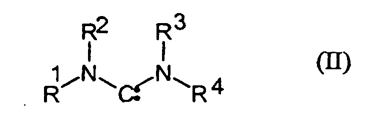 worin
- die Reste R¹, R², R³ und R⁴: die oben genannten Bedeutungen aufweisen.

Weiterhin bevorzugt steht L für gleiche oder unabhängig voneinander verschiedene neutrale Zweielektronen-Donor-Liganden aus der Gruppe der Verbindungen der allgemeinen Formeln (III) und (IV) 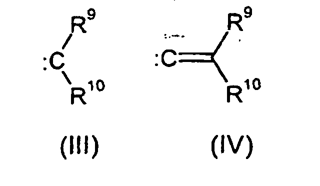 worin
- die Reste R⁹ und R¹⁰: gleich oder verschieden sind und für Wasserstoff oder/und für Kohlenwasserstoffgruppen stehen, wobei es sich bei den Kohlenwasserstoffgruppen um gleiche oder verschiedene, geradkettige, verzweigte oder cyclische Reste aus der Gruppe Alkylreste mit 1 bis 50 Kohlenstoffatomen, Alkenylreste mit 2 bis 50 Kohlenstoffatomen, Alkinylreste mit 2 bis 50 Kohlenstoffatomen, Arylreste mit 6 bis 30 Kohlenstoffatomen und Silylreste handelt, in denen gegebenenfalls mindestens ein Wasserstoffatom durch eine eine Alkyl-, Aryl-, Alkenyl-, Alkinyl-, Metallocenyl-, Halogen-, Nitro-, Nitroso-, Hydroxy-, Alkoxy-, Aryloxy-, Amino-, Amido-, Carboxyl-, Carbonyl-, Thio- oder/und Sulfonylgruppe ersetzt ist.

Steht L in den erfindungsgemäßen Verbindungen der allgemeinen Formel (I) für einen Teil eines cyclischen Systems, so handelt es sich bei dem cyclischen System bevorzugt um ein cyclisches Dien, bevorzugt um ein Dien mit 5 bis 10 Kohlenstoffatomen, ganz besonders bevorzugt um 1,5-Cyclooctadien.

Steht L in den erfindungsgemäßen Verbindungen der allgemeinen Formel (I) für gleiche oder unabhängig voneinander verschiedene anionische Liganden, so handelt es sich dabei vorzugsweise um Liganden aus der Reihe Halogenide, Pseudohalogenide, Hydroxide, Alkoxide, Thiolate, Carboxylate und Sulfonate, wobei unter den Pseudohalogeniden Cyanid, Rhodanid, Cyanat, Isocyanat, Thiocyanat und Isothiocyanat bevorzugt sind.

In einer bevorzugten Ausführungsform der erfindungsgemäßen Verbindungen der allgemeinen Formel (I) steht M für Rhodium oder Iridium und n für 3, wobei L¹, L² und L³ gleich oder/und voneinander unabhängig verschieden sind und/oder Teil eines cyclischen Systems sind, wobei
- L¹: für einen anionischen Liganden aus der Reihe Halogenid, Alkoxid, Carboxylverbindung, Thiolat und Pseudohalogenid steht oder für einen neutralen Zweielektronen-Donoren aus der Reihe Alkene, Alkine, Phosphane, Amine, Imine, Phosphite, Stibine, Arsine, CO, Carbonylverbindungen, Nitrile, Alkohole, Ether, Thiole und Thioether steht und
- L² und L³: gleich oder unabhängig voneinander verschieden sind und für neutrale Zweielektronen-Donor-Liganden aus der Reihe Alkene, Alkine, Phosphane, Amine, Imine, Phosphite, Stibine, Arsine, CO, Carbonylverbindungen, Nitrile, Alkohole, Ether, Thiole und Thioether stehen, oder in bevorzugter Weise Teil eines cyclischen Diens sind, besonders bevorzugt stehen L² und L³ gemeinsam für 1,5-Cyclooctadien.

In einer besonders bevorzugten Ausführungsform der erfindungsgemäßen Verbindungen der allgemeinen Formel (I) steht M für Rhodium oder Iridium und n für 3, wobei L² und L³ gemeinsam für ein cyclisches Dien, ganz besonders bevorzugt für 1,5-Cyclooctadien stehen und L¹ für Halogenid oder CO steht, wobei die Ladung von ein- oder dreiwertigem Rhodium oder Iridium kompensiert wird und die Koordinationszahlen 4, 5 oder 6 resultieren.

In einer weiteren bevorzugten Ausführungsform der erfindungsgemäßen Verbindungen der allgemeinen Formel (I) steht M für Ruthenium oder Osmium und n für 4, wobei
- L¹ und L²: gleich oder unabhängig voneinander verschieden sind und für einen anionischen Liganden aus der Reihe Halogenid, Alkoxid, Carboxylverbindung, Thiolat und Pseudohalogenid stehen, wobei unter den Pseudohalogeniden Cyanid, Rhodanid, Cyanat, Isocyanat, Thiocyanat und Isothiocyanat bevorzugt sind,
- L²: für einen Carbenliganden der allgemeinen Formel (III) oder (IV) und
- L⁴: für einen neutralen Zweielektronen-Donor-Ligand aus der Reihe Amine, Imine, Phosphane, Phosphite, Stibine, Arsine, CO, Carbonylverbindungen, Nitrile, Alkohole, Thiole, Ether und Thioether oder für einen neutralen Zweielektronen-Donor aus der Gruppe der N-heterocyclischen Carbene der allgemeinen Formel A - E oder für einen Diaminocarben-Ligand der allgemeinen Formel (II) steht.

Steht L⁴ für einen Diaminocarben-Liganden der allgemeinen Formel (II), so sind R¹ und R² und/oder R³ und R⁴ bevorzugt Teil eines cyclischen Systems, welches gleich oder verschieden ist, wobei das cyclische System aus einem Kohlenstoffgerüst mit 2 bis 20 Kohlenstoffatomen, bevorzugt 2 bis 10 Kohlenstoffatome, und einem Stickstoffatom in Formel (II) besteht, wobei die Kohlenstoffatome von R¹ und R² und/oder R³ und R⁴ in Formel (II) nicht mit eingerechnet werden und wobei gegebenenfalls mindestens ein Wasserstoffatom im Cyclus durch eine funktionelle Gruppe ersetzt ist.

In einer weiteren besonders bevorzugten Ausführungsform der erfindungsgemäßen Verbindungen der allgemeinen Formel (I) steht M für Ruthenium oder Osmium und n für 4, wobei L¹ und L² gleich oder verschieden sind und für Chlorid, Bromid oder Iodid stehen, L³ für einen Carbenliganden der allgemeinen Formel (III) oder (IV) steht und L⁴ für ein Phosphan steht, vorzugsweise für Trimethylphosphan, Tricyclohexylphosphan oder Triphenylphosphan, und zwar dergestalt, dass die Ladung des zwei-, vier oder sechswertigen Rutheniums oder Osmiums kompensiert wird und die Koordinationszahlen 4, 5 oder 6 resultieren.

In einer weiteren bevorzugten Ausführungsform der erfindungsgemäßen Verbindungen der allgemeinen Formel (I) steht M für Cr und n für 4 wobei die Liganden L¹ bis L⁴ gleich oder verschieden sind und für neutralen Zweielektronen-Donor-Liganden wie beispielsweise CO, Phosphan, Phosphit, Stibin, Arsin, Alken, Alkin, Amin, Imin, Carbonylverbindungen, Nitril, Alkohol, Ether, Thiol, Thioether oder Nitril stehen, ganz besonders bevorzugt sind L¹ bis L⁴ gleich und stehen für CO.

Prinzipiell stehen verschiedene literaturbekannte Methoden zur Synthese der erfmdungsgemäßen Verbindungen zur Verfügung, die bereits zur Herstellung von Übergangsmetallkomplexen mit gesättigten oder ungesättigten N-heterocyclischen Carbenliganden etabliert sind. Dabei handelt es sich um die Umsetzung geeigneter Übergangsmetallkomplex-Vorstufen mit den freien Liganden des Typs R¹R²N-C-NR³R⁴, welche aus den entsprechenden Formamidiniumsalzen [R¹R²N-CH=NR³R⁴]⁺X⁻ hergestellt werden können oder mit den Formamidiniumsalzen selbst (Salzmetathese). Diese Methoden wurden in der vorliegenden Erfindung erstmals auf Diaminocarben-Liganden erfolgreich angewandt. Die betreffenden erfindungsgemäßen Verbindungen der Formel (I) waren nach dem Stand der Technik nicht als stabile Systeme zu erwarten.

Zur Herstellung der erfindungsgemäßen Verbindungen der Formel (I) in einer Ligandenaustauschreaktion via freies Carben wird vorzugsweise der freie Carbenligand der allgemeinen Formel (II) mit geeigneten Übergangsmetallkomplexen umgesetzt, wobei das freie Carben aus dem Formamidiniumsalz durch Deprotonierung mit starken Basen wie beispielsweise Lithiumdiisopropylamid (Angew. Chem. 1996, 108, 1211; Angew. Chem Int. Ed. Engl. 1996, 35, 1121; Tetrahedron 1999,55 14523-14534), besonders bevorzugt jedoch nach der in DE 196 10 908 A1 beschriebenen "Ammoniak-Methode" hergestellt wird.

Zur Herstellung der erfindungsgemäßen Verbindungen der Formel (I) durch Salzmetathese werden die Formamidiniumsalze mit geeigneten Übergangsmetallkomplexen umgesetzt.

Es ist aber auch möglich, das Carben der allgemeinen Formel (II) intermediär aus dem Alkoholat-Addukt des Formamidiniumsalzes mit Alkalimetallalkoholaten, vorzugsweise mit Kalium-tert.-butanolat durch kurzes Erwärmen in situ zu erzeugen, welches in Anwesenheit von entsprechenden Übergangsmetallkomplexen durch Ligandenaustauschreaktion zu den erfindungsgemäßen Verbindungen reagiert (WO 00/71554 A2 und WO 00/15339 A1).

Die Herstellung der erfindungsgemäßen Verbindungen der Formel (I) erfolgte vorzugsweise nach folgenden Methoden:

### a) Ligandenaustauschreaktion via freies Carben:

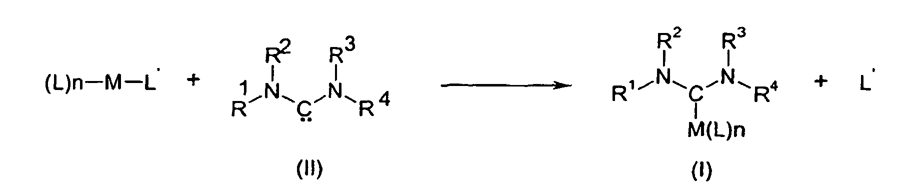

L' ist vorzugsweise ein neutraler Austauschligand wie beispielsweise Nitril oder Phosphan. Die Ligandenaustauschreaktion wird vorzugsweise für die Herstellung der erfindungsgemäßen, Verbindungen der Formel (I), wobei M für Ruthenium oder Osmium steht, eingesetzt.

### b) Salzmetathese:

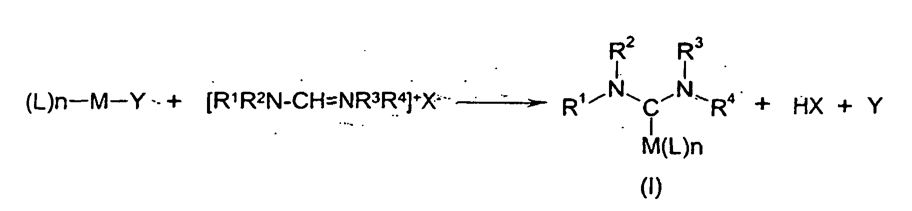

Y ist vorzugsweise ein neutraler oder anionischer Austauschligand , beispielsweise Amin, Halogenid, Acetat, Acetylacetonat oder Alkoxid. Die Salzmetathese wird vorzugsweise für die Herstellung der erfindungsgemäßen Verbindungen der Formel (I), wobei M für Rhodium oder Iridium steht, eingesetzt.

### c) Alkoholat-Addukt-Methode (Erzeugung des Carbens in situ):

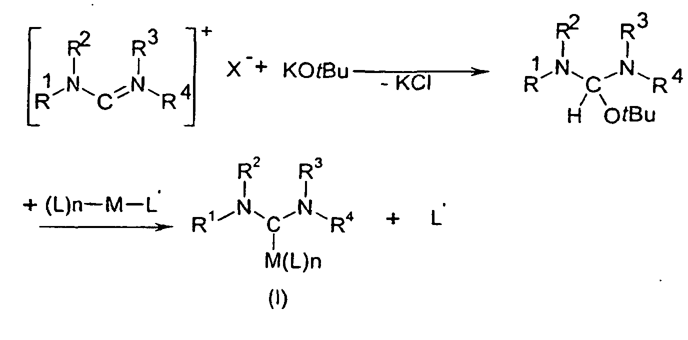

L' ist vorzugsweise ein neutraler Austauschligand wie beispielsweise Nitril oder Phosphan. Die Ligandenaustauschreaktion wird vorzugsweise für die Herstellung der erfmdungsgemäßen Verbindungen der Formel (I), wobei M für Ruthenium oder Osmium steht, eingesetzt.

Die erfindungsgemäßen Verbindungen werden vorzugsweise als Katalysatoren in organisch-chemischen Synthesereaktionen eingesetzt, beispielsweise als Katalysatoren für die Hydrosilylierung, Hydroformylierung, Olefin-Metathese, Alkin-Metathese, Heck-Kupplung, Suzuki-Kupplung, Sonogashira-Kupplung, Cyclopropanierung oder Grignard-Kreuzkupplung. Bevorzugt werden erfindungsgemäße Verbindungen der Formel (I), wobei M für Ruthenium oder Osmium steht, in der Olefin-Metathese eingesetzt. Weiterhin werden bevorzugt erfindungsgemäße Verbindungen der Formel (I), wobei M für Rhodium oder Iridium steht, in der Hydrosilylierung eingesetzt. Die erfmdungsgemäßen Verbindungen können dabei sowohl als homogene als auch als heterogene Katalysatoren eingesetzt werden. Die letztgenannte Ausführungsform wird vorzugsweise dadurch realisiert, dass die erfmdungsgemäßen Verbindungen an eine feste Phase, beispielsweise an einen polymeren Träger, gebunden werden. Die Anbindung der erfindungsgemäßen Verbindungen an die feste Phase erfolgt vorzugsweise über einen oder mehrere Liganden L, vorzugsweise über einen Liganden L aus der Gruppe der N-heterocyclischen Carbene der allgemeinen Formel A - E und/oder über einen Diaminocarben-Liganden der allgemeinen Formel (II).

Die erfindungsgemäßen Verbindungen sind hochaktive, besonders kostengünstig und in guten Ausbeuten zu synthetisierende Katalysatoren. Die erfindungsgemäßen Verbindungen zeichnen sich weiterhin durch ihre mögliche Variationsvielfalt in der Ligandensphäre aus, speziell durch Variation der Diamino-Carbenliganden R¹R²N-C-NR³R⁴ kann die Katalysatoraktiviät und -selektivität gesteuert werden.

Der Vorteil dieser erfindungsgemäßen Komplexverbindungen verglichen mit phosphanhaltigen oder/und gemischten Phosphan/ N-heterocyclischen carbenhaltigen Komplexen liegt in der kostengünstigen Herstellung der Diamino-Carbenliganden. Die Stabilität, Selektivität und Aktivität der Katalysatoren kann deutlich gegenüber bestehenden Systemen gesteigert werden.

### Beispiele

Die folgenden Beispiele belegen die Erfindung, schränken sie aber nicht in ihrer Breite ein.

### Beispiel 1: Vorstufen zur Herstellung der erfindungsgemäßen Komplexverbindungen

Tetramethylformamidiniumchlorid ist kommerziell erhältlich. Tetraisopropylformamidinium-chlorid wurde gemäß Angew. Chem. 1996, 108, 1211 und Angew. Chem. Int. Ed. Engl., 1996, 35, 1121 synthetisiert.

### a) Herstellung von Dipiperidylformamidiniumchlorid:

6,08 g (0,05 mol) Piperidiniumhydrochlorid, 4,26 g (0,05 mol) Piperidin und 7,41 g (0,05 mol) Triethylameisensäureorthoester wurden in 25 ml Ethanol 4 Stunden unter Rückfluss erhitzt. Nach Abkühlen über Nacht wurden die flüchtigen Bestandteile im Hochvakuum entfernt. Der Rückstand wurde mit 20 ml THF (Tetrahydrofuran) gewaschen und anschließend mit 100 ml einer Mischung aus 20 Vol.-% Ethanol und 80 Vol.-% Ethylacetat extrahiert. Das Extrakt wurde über Magnesiumsulfat getrocknet. Nach Entfernen des Lösungsmittels im Hochvakuum erhielt man Dipiperidylformamidiniumchlorid als leicht gelblichen, stark hygroskopischen Feststoff.

¹H-NMR (CDCl₃, 25 °C): δ = 7.94 (1H, s, NC*H*N), 3.03, 3.36 (8H, m, NC*H*₂), 1.5 - 0.91 (m, 12H, restliche CH₂).
¹³C-NMR (CDCl₃, 25 °C): δ = 154.3 (N*C*HN), 51.9, 44.4 (N*C*H₂), 25.5, 22.7, 22.2 (NCH₂*C*H₂*C*H₂*C*H₂CH₂).

### b) Herstellung von Dipiperidylformamidiniumtetrafluoroborat

5 ml (0,01 mol) Piperidin, 2,6 g (0,025 mol) Ammoniumtetrafluoroborat und 40 ml Triethylameisensäureorthoester wurden 2 Stunden unter Rückfluss erhitzt. Entstehendes Ethanol wurde abdestilliert. Dabei bildeten sich zwei Phasen. Nach Abkühlen wurde die untere gelbe Phase in Methylenchlorid aufgenommen und über Magnesiumsulfat getrocknet. Nach Entfernen des Lösungsmittels im Hochvakuum erhielt man Dipiperidylformamidinium-tetrafluoroborat als leicht gelblichen, stark hygroskopischen Feststoff.

### Beispiel 2: Synthese der freien Carbene

### a) Deprotonierung mit NaH/NH₃(1) (Ammoniak-Methode)

10 mmol des entsprechenden Formamidiniumchlorids bzw. -tetrafluoroborats wurden in etwa 20 ml THF vorgelegt. Nach dem Aufkondensieren von etwa 20 ml Ammoniak wurden 11 mmol NaH zugegeben. Im Laufe von etwa 2 h bildete sich eine leicht gelbliche Lösung. Anschließend wurde zunächst der Ammoniak abgedampft, danach das THF im Hochvakuum entfernt und das freie Carben mit Hexan extrahiert. Die so erhaltene Lösung kann direkt weiter eingesetzt werden.

### b) Deprotonierung der Formamidiniumsalze mit Lithiumdiisopropylamid

Eine Lösung von 10 mmol des entsprechenden Formamidiniumchlorides oder -tetrafluoroborats in 15 ml THF wurde bei -78 °C langsam zu einer Lösung von 10 mmol Lithiumdiisopropylamid in 15 ml THF getropft. Binnen zwei Stunden wurde langsam auf Raumtemperatur erwärmt. Dabei bildete sich eine klare, leicht gelbliche Lösung. Das Lösungsmittel wurde im Hochvakuum entfernt. Es wurde dreimal mit je 15 ml Toluol extrahiert. Die so erhaltene Lösung wird direkt weiter eingesetzt.

### Beispiel 3: Synthese der erfindungsgemäßen Komplexverbindungen

### a) Synthese der Rh/Ir-Verbindungen durch Ligandenaustauschreaktion

### I) Chloro(η⁴-1,5-cyclooctadien)(bis(diisopropylaminocarben)rhodium(I)

Eine Lösung von Bis(diisopropylamino)carben (340 mg, 1,71 mmol, 2 eq) in THF (20 ml) wurde tropfenweise zu einer Lösung von Bis[µ-chloro(1,5-cyclooctadiene)-rhodium] (421 mg, 0,85 mmol, 1 eq) in 40 ml THF gegeben. Die Farbe änderte sich von hellgelb nach dunkelgelb. Nach 30 min Rühren bei Raumtemperatur wurde das Lösungsmittel im Vakuum entfernt. Der Niederschlag wurde mit eiskaltem Diethylether (2 x 10 ml) gewaschen und im Vakuum getrocknet. Ausbeute: 533 mg, 71 %.

¹H NMR (400 MHz, CDCl₃, 20 °C): δ = 5.16 (2 H, cod_{vinyl}), 4.73 (1 H, cod_{vinyl}), 4.09 (4H, NCH), 3.09 (1 H, cod_{vinyl}), 2.35 (4 H, cod_{allyl}), 1.74 - 1.17 (m, 28 H, cod_{allyl}, CH₃).
¹³C {¹H} NMR (100.1 MHz, CDCl₃, 20 °C): δ = 233.6 (NCN, d, ¹J(C-Rh) = 67.8 Hz), 97.5, 67.3, 32.5, 30.9, 28.6 (cod), 56.4 (NCH), 23.8 (CH₃).
MS (CI) *m*/*z* = 458 (M⁺, korrektes Isotopenmuster), 423 (M - Cl, korrektes Isotopenmuster), 314 (M - Cl - cod, korrektes Isotopenmuster).
Elementaranalyse: (C₂₁H₄₀ClN₂Rh, 458.92): berechnet: C 54.96, H 8.78, N 6.10; gefunden: C 54.90, H 8.81, N 6.17.

### II) Chloro(η⁴-1,5-cyclooctadien)(bisdiisopropylamino-carben)iridium(I)

Analog der Synthese des Rhodiumkomplexes wurde Bis[µ-chloro(1,5-cyclooctadien)-iridium(I)] (340 mg, 1,00 mmol, 1 eq) mit Bis(diisopropylamino)carben (200 mg, 1,00 mmol, 2 eq) zur gewünschten Komplexverbindung umgesetzt. Ausbeute: 746 mg, 68 %.

¹H NMR (400 MHz, CDCl₃, 20 °C): δ = 5.13 (2 H, cod_{vinyl}), 4.73 (2 H, cod_{vinyl}), 3.85 (4 H, NCH), 2.95 (4 H, cod_{allyl}), 2.11 (m, 4 H, cod_{allyl}), 1.58-1.17 (m, 24 H, CH₃).
¹³C {¹H} NMR (100.1 MHz, CDCl₃, 20 °C): δ = 225.6 (NCN), 82.1, 67.9, 33.2, 31.9, 29.2 (cod), 51.1 (NCH), 23.8 (CH₃).
MS (CI) *m*/*z* = 548 (M⁺, korrektes Isotopenmuster).
Elementaranalyse: (C₂₁H₄₀ClN₂Ir, 548.23), berechnet: C 46.01, H 7.35, N 5.11; gefunden: C 46.09, H 7.41, N 5.07.

### b) Synthese der Rh/Ir-Komplexe durch Salzmetathese

### I) Chloro(η⁴-1,5-cyclooctadien)(bis(diisopropylaminocarben)rhodium(I)

Eine 1 M Lösung von Natriummethanolat (2,48 ml) wurde tropfenweise zu einer Suspension von Bis[µ-chloro(1,5-cyclooctadien)-rhodium(I)] (300 mg, 0,61 mmol, 1 eq) in Ethanol (30 ml) gegeben. Innerhalb von 5 min wechselte die Farbe auf Grund der Bildung von Bis[µ-ethoxy(1,5-cyclooctadien)-rhodium(I)] nach zitronengelb. Nach weiteren 10 min Rühren bei Raumtemperatur wurde das Bis(diisopropyl)-formamidiniumchlorid (400 mg, 1,22 mmol, 2 eq) zugegeben. Die Farbe wechselte nach dunkelgelb. Nach weiteren 30 min Rühren bei Raumtemperatur wurden die flüchtigen Bestandteile im Vakuum entfernt und das Rohprodukt mit eiskaltem Diethylether (2 x 10 ml) gewaschen und getrocknet. Ausbeute: 531 mg, 68 %.

### II) Chloro(η⁴-1,5-cyclooctadien)(bisdiisopropylamino-carben)iridium(I)

Analog zu Beispiel 3b, I) wurden Bis[µ-chloro(1,5-cyclooctadien)-iridium(I)] (333 mg, 0,61 mmol, 1 eq), 1M Natriummethanolat-Lösung (2.48 ml) und Bis(diisopropyl)formamidinium chloride (400 mg, 2 eq) zur gewünschten Iridium-Verbindung in 71 % Ausbeute umgesetzt.

### c) Synthese der Rh/Ir-Komplexe via Alkoholat-Addukt

### I) Chloro(η⁴-1,5-cyclooctadien)(bis(diisopropylaminocarben)rhodium(I)

Eine Lösung von Kalium-*tert*-butanolat (191 mg, 1,70 mmol, 2,8 eq) in THF (20 ml) wurde zu einer Suspension von Bis(diisopropyl)formamidiniumchlorid (280 mg, 1,70 mmol, 2,8 eq) in THF (30 ml) bei Raumtemperatur getropft, wobei sich das Formamidiniumchlorid langsam löste und die Farbe nach leicht gelb wechselte. Nach 30 min Rühren bei Raumtemperatur wurden Bis[µ-chloro(1,5-cyclooctadien)-rhodium(I)] (300 mg, 0,61 mmol, 1 eq) und Toluol (30 ml) zugegeben. Die Reaktionslösung wurde 1 h bei 80°C gerührt. Danach wurden die flüchtigen Bestandteile im Vakuum entfernt und das gewünschte Produkt mit 60 ml Hexan extrahiert. Nach dem Trocknen wurde der Komplex in 73% Ausbeute erhalten.

### II) Chloro(η⁴-1,5-cyclooctadien)(bis(diisopropylaminocarben)iridium(I)

Analog zu Beispiel 3c, I) wurden Bis[µ-chloro(1,5-cyclooctadien)-iridium(I)] (333 mg, 0,61 mmol, 1 eq), KO*t*Bu (191 mg, 1,70 mmol, 2,8 eq) und Bis(diisopropyl)formamidiniumchlorid (280 mg, 1,70 mmol, 2,8 eq) zur gewünschten Ir-Verbindung in 66% Ausbeute umgesetzt.

### c) Synthese eines Cr-Komplexes durch Ligandenaustauschreaktion

### I) Tetracarbonyl(bisdiisopropylaminocarben)chrom(0)

Eine Lösung von 390 mg (1,6 mmol) Cr(CO)₆ in 50ml THF wurde bei Raumtemperatur 3,5 h unter Rühren mit einer Quecksilberdampflampe (150W) bestrahlt. 200 mg (0,94 mmol) Bis(diisopropylamino)carben wurden in 5 ml Tetrahydrofuran gelöst und unter heftigem Rühren zu der orangefarbenen Lösung getropft. Nach weiteren 30 min Rühren bei Raumtemperatur wechselte die Farbe der Reaktionslösung nach rotbraun. Das Lösemittel wurde im Vakuum abdestilliert und überschüssiges Cr(CO)₆ wurde durch Sublimation im Hochvakuum entfernt. Der braune Rückstand wurde dreimal mit je 20 mL Diethylether extrahiert, die vereinigten Extrakte auf 5 ml konzentriert und dann über eine 50 cm x 1.0 cm lange Kieselgelsäule mit Hexan chromatographiert. Nachdem eine schwach gelbe Zone mit Hexan/Diethylether (2:1) eluiert wurde (enthält hauptsächlich Cr(CO)₆), konnte das Produkt mit Diethylether als dunkelgelbe Bande eluiert werden. Die Produktfraktionen wurden im Vakuum auf 1-2 ml konzentriert und nach der Zugabe von 5-10 ml Hexan fiel der Chromkomplex bei 0°C in Form von dunkelgelben Kristallen aus, die aus Diethylether / Hexan umkristallisiert werden konnten. Ausbeute: 80 mg (23 %)

¹H-NMR (400.13 MHz, C₆D₆): δ 0.942 (br. CH₃, 24H), 2.961 (br, CH, 4H)
IR (Hexan, cm⁻¹): ν = 2003.8 (m, CO); 1918.4 (w, CO); 1896.3 (s, CO); 1870.1 (m, CO)
Elementaranalyse: C₁₇H₂₈CrN₂O₄ C: 54.24; H: 7.50, N: 7.44 (berechnet); C: 54.85; H: 7.39; N: 6.94 (gefunden).

In analoger Weise wurden aus Mo(CO)₆ und W(CO)₆ die entsprechenden Molybdän- bzw. Wolfram-Verbindungen synthetisiert.

### Beispiel 4: Herstellung von Dicarbonylchloro(bis(diisopropylamino)-carben)rhodium(I) durch Ligandenaustauschreaktion

Kohlenmonoxid wurde 30 min durch eine gelbe Lösung von Chloro(η⁴-1,5-cyclooctadien)(bis(diisopropylaminocarben)rhodium(I) (300 mg, 0,65 mmol) in eine 1:1 Mischung von THF und Toluol (35 ml) geleitet. Nachdem sich die Reaktionslösung bräunlich gefärbt hatte, wurde das Lösungsmittel im Vakuum entfernt und das Rohprodukt mit n-Pentan gewaschen. Ausbeute: 68 %.
¹H NMR (400 MHz, CDCl₃, 20 °C): δ = 4.11 (4H, NCH), 1.36 (24H, CH₃)
¹³C {¹H} NMR (100.1 MHz, CDCl₃, 20 °C): δ = 221.7 (NCN, d, ¹J(C-Rh) = 64 Hz), 186.9 (CO, d, ¹J(C-Rh) = 74 Hz), 56.1 (NCH), 23.5 (CH₃)
MS (CI) *m*/*z* = 406 (M⁺, korrektes Isotopenmuster).
IR (KBr, cm⁻¹): ν = 2056 (s, CO), 1985 (s, CO).
Elementaranalyse: (C₁₅H₂₈ClN₂O₂Rh, 406.76): berechnet: C 44.29, H 6.93, N 6.88; gefunden: C 44.32, H 6.89, N 6.91.

### Beispiel 5: Hydrosilylierung von Acetophenon

0,61 ml einer Edukt-Stammlösung (bestehend aus 0,8 ml Acetophenon, 1,3 ml Diphenylsilan und 300 mg Ferrocen als interner Standard gelöst in 4 ml d₈-THF) wurden zusammen mit 1 Mol-% Chloro(η⁴-1,5-cyclooctadien)(bis(diisopropylaminocarben)rhodium(I) als Katalysator (als Stammlösung, in 0,2 ml d₈-THF) in ein NMR-Rohr gegeben. Nach kräftigem Durchschütteln wurde sofort mit der Aufnahme der NMR-Kinetik begonnen, wobei alle 10 Minuten ein ¹H-NMR-Spektrum aufgenommen wurde. Der Umsatz wurde durch die Integration der relevanten Protonensignale bestimmt. Die Ergebnisse sind in Tabelle 1 widergegeben.

**Tabelle 1:**

| Hydrosilylierung von Acetophenon mit unter Einsatz verschiedener Katalysatoren | | | | |
|---|---|---|---|---|
| | **Umsatz Acetophenon [%]** | | | |
| **Zeit [min]** | **KD2** | **KD1** | **iPr-BIM** | **Cy-IM** |
| 0,0 | 0,0000 | 0,0000 | 0,0000 | 0,0000 |
| 1,5 | 2,1083 | 5,3043 | 16,5262 | 49,7128 |
| 11,5 | 10,2384 | 19,7991 | 22,2451 | 63,8878 |
| 21,5 | 18,3888 | 27,1516 | 36,1491 | 64,2819 |
| 31,5 | 22,2013 | 40,4057 | 51,9386 | 64,4957 |
| 41,5 | 26,4179 | 50,1740 | 62,4524 | 64,6827 |
| 51,5 | 33,0257 | 57,5527 | 68,3399 | 65,2572 |
| 61,5 | 38,2527 | 63,9139 | 72,6104 | 65,6246 |
| 71,5 | 43,7828 | 68,4960 | 75,0390 | 66,2191 |
| 81,5 | 48,1140 | 72,3495 | 77,5176 | 66,6333 |
| 91,5 | 53,3275 | 75,7369 | 79,2908 | 66,8270 |
| 101,5 | 57,5778 | 78,5466 | 80,7767 | 67,2545 |
| 111,5 | 61,1949 | 80,5160 | 82,0191 | 67,2612 |
| 121,5 | 63,8488 | 81,5992 | 82,8307 | 67,4349 |
| 131,5 | 66,3142 | 82,0259 | 83,6486 | 67,5618 |
| 141,5 | 66,6038 | 82,4526 | 84,1668 | 67,7355 |
| 151,5 | 68,8199 | 82,6626 | 84,6663 | 67,9225 |
| 161,5 | 70,5914 | 82,7677 | 85,0846 | 67,8156 |
| 171,5 | 72,6122 | 82,9121 | 85,3843 | 68,0227 |
| 181,5 | 72,1137 | 83,1287 | 85,7402 | 68,1496 |
| 191,5 | 74,4308 | 83,2994 | 85,8900 | 68,1764 |
| 201,5 | 76,6941 | 83,5554 | 86,0586 | 68,3901 |

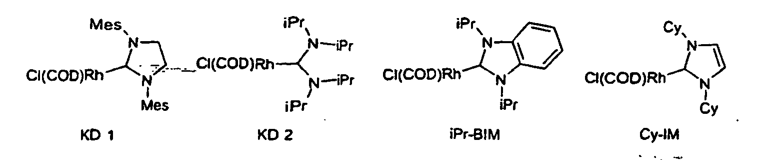

wobei COD für einen 1,5-Cyclooctadienylrest steht, iPr für einen Isopropylrest steht, Cy für einen Cyclohexylrest steht und Mes für einen Mesitylrest steht.

## Patentansprüche

1. Verbindungen der allgemeinen Strukturformel (I), 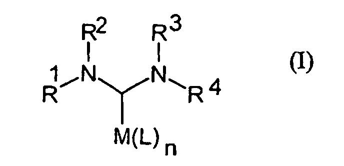 worin
M für ein Übergangsmetall der 6. bis 8. Nebengruppe des Periodensystems steht,
die Reste R¹, R², R³ und R⁴ gleich oder verschieden sind und für Wasserstoff oder/und für Kohlenwasserstoffgruppen stehen, wobei es sich bei den Kohlenwasserstoffgruppen um gleiche oder verschiedene, geradkettige, verzweigte oder cyclische Resten aus der Gruppe Alkylreste mit 1 bis 50 Kohlenstoffatomen, Alkenylreste mit 2 bis 50 Kohlenstoffatomen, Alkinylreste mit 2 bis 50 Kohlenstoffatomen und Arylreste mit 6 bis 30 Kohlenstoffatomen, in denen gegebenenfalls mindestens ein Wasserstoffatom durch eine funktionelle Gruppen ersetzt ist, handelt und/oder
R¹ und R² und/oder R³ und R⁴ Teil eines cyclischen Systems sind, welches gleich oder verschieden ist, wobei das cyclische System aus einem Kohlenstoffgerüst mit 2 bis 20 Kohlenstoffatomen und einem Stickstoffatom in Formel (I) besteht, wobei die Kohlenstoffatome von R¹ und R² und/oder R³ und R⁴ in Formel (I) nicht mit eingerechnet werden und wobei gegebenenfalls mindestens ein Wasserstoffatom im Cyclus durch eine funktionelle Gruppe ersetzt ist und/oder gegebenenfalls mindestens ein Kohlenstoffatom des Cyclus durch ein Heteroatom aus der Reihe S, P, O oder N ersetzt ist, und/oder
R¹ und/oder R² und/oder R³ und/oder R⁴ über eine Brücke von 1 bis 20 Kohlenstoffatomen, wobei die Kohlenstoffatome von R¹, R² R³, und R⁴ nicht mit eingerechnet werden, mit einem der Liganden L verknüpft sind, und
L für gleiche oder unabhängig voneinander verschiedene neutrale Zweielektronen-Donoren stehen und/oder Teile eines cyclischen Systems sind und/oder für anionische Liganden stehen, und
n für eine ganze Zahl steht.

2. Verbindungen nach Anspruch 1, wobei L für gleiche oder unabhängig voneinander verschiedene neutrale Zweielektronen-Donoren aus der Reihe Amine, Imine, Phosphane, Phosphite, Stibine, Arsine, CO, Carbonylverbindungen, Nitrile, Alkohole, Thiole, Ether, Thioether und Pyridine und/oder
für gleiche oder unabhängig voneinander verschiedene neutrale Zweielektronen-Donoren aus der Reihe der N-heterocyclischen Carbene der allgemeinen Formeln (A - E) 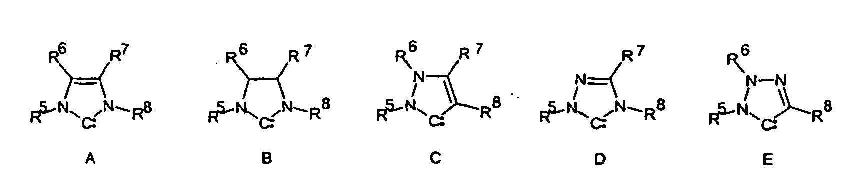 worin
die Reste R⁵, R⁶, R⁷ und R⁸ gleich oder verschieden sind und für Wasserstoff oder/und für Kohlenwasserstoffgruppen stehen, wobei es sich bei den Kohlenwasserstoffgruppen um gleiche oder verschiedene, geradkettige, verzweigte oder cyclische Reste aus der Gruppe Alkylreste mit 1 bis 50 Kohlenstoffatomen, Alkenylreste mit 2 bis 50 Kohlenstoffatomen, Alkinylreste mit 2 bis 50 Kohlenstoffatomen und Arylreste mit 6 bis 30 Kohlenstoffatomen, in denen gegebenenfalls mindestens ein Wasserstoffatom durch eine funktionelle Gruppe ersetzt ist, handelt und/oder
die Reste R⁶ und R⁷ für gleiche oder unabhängig voneinander verschiedene Halogen-, Nitro-, Nitroso-, Alkoxy-, Aryloxy-, Amido-, Carboxyl-, Carbonyl-, Thio- oder/und Sulfonylgruppen stehen, und/oder
für gleiche oder unabhängig voneinander verschiedene neutrale Zweielektronen-Donoren aus der Reihe der Diaminocarben-Liganden der allgemeinen Formel (II), 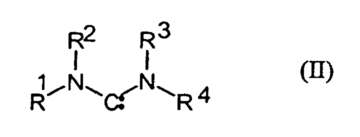 worin
die Reste R¹, R², R³ und R⁴ die in Anspruch 1 genannten Bedeutungen aufweisen und/oder
für gleiche oder unabhängig voneinander verschiedene neutrale Zweielektronen-Donor-Liganden aus der Reihe der Gruppe der Verbindungen der allgemeinen Formeln (III) und (IV) 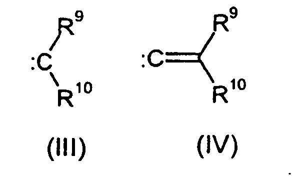 worin
die Reste R⁹ und R¹⁰ gleich oder verschieden sind und für Wasserstoff oder/und für Kohlenwasserstoffgruppen stehen, wobei es sich bei den Kohlenwasserstoffgruppen um gleiche oder verschiedene, geradkettige, verzweigte oder cyclische Resten aus der Gruppe Alkylreste mit 1 bis 50 Kohlenstoffatomen, Alkenylreste mit 2 bis 50 Kohlenstoffatomen, Alkinylreste mit 2 bis 50 Kohlenstoffatomen, Arylreste mit 6 bis 30 Kohlenstoffatomen und Silylreste handelt, in denen gegebenenfalls mindestens ein Wasserstoffatom durch eine eine Alkyl-, Aryl-, Alkenyl-, Alkinyl-, Metallocenyl-, Halogen-, Nitro-, Nitroso-, Hydroxy-, Alkoxy-, Aryloxy-, Amino-, Amido-, Carboxyl-, Carbonyl-, Thio- oder/und Sulfonylgruppe ersetzt ist, stehen.

3. Verbindungen nach Anspruch 1, wobei L für gleiche oder unabhängig voneinander verschiedene anionische Liganden aus der Reihe Halogenide, Pseudohalogenide, Hydroxide, Alkoxide, Thiolate, Carboxylate und Sulfonate steht, wobei unter den Pseudohalogeniden Cyanid, Rhodanid, Cyanat, Isocyanat, Thiocyanat und Isothiocyanat bevorzugt sind.

4. Verbindungen nach Anspruch 1, wobei L für ein cyclisches Dien, vorzugsweise für ein Dien mit 5 bis 10 Kohlenstoffatomen steht.

5. Verbindungen nach Anspruch 1, wobei M für Rhodium oder Iridium und n für 3 steht und wobei L¹, L² und L³ gleich oder/und voneinander unabhängig verschieden sind und/oder Teil eines cyclischen Systems sind, wobei
L¹ für einen anionischen Liganden aus der Reihe Halogenid, Alkoxid, Carboxylverbindung, Thiolat und Pseudohalogenid steht oder für einen neutralen Zweielektronen-Donoren aus der Reihe Alkene, Alkine, Phosphane, Amine, Imine, Phosphite, Stibine, Arsine, CO, Carbonylverbindungen, Nitrile, Alkohole, Ether, Thiole und Thioether steht und
L² und L³ gleich oder unabhängig voneinander verschieden sind und für neutrale Zweielektronen-Donoren aus der Reihe Alkene, Alkine, Phosphane, Amine, Imine, Phosphite, Stibine, Arsine, CO, Carbonylverbindungen, Nitrile, Alkohole, Ether, Thiole und Thioether stehen, oder in bevorzugter Weise Teil eines cyclischen Diens sind, besonders bevorzugt stehen L² und L³ gemeinsam für 1,5-Cyclooctadien.

6. Verbindungen nach Anspruch 1, wobei M für Ruthenium oder Osmium und n für 4 steht und wobei
L¹ und L² gleich oder unabhängig voneinander verschieden sind und für einen anionischen Liganden aus der Reihe Halogenid, Alkoxid, Carboxylverbindung, Thiolat und Pseudohalogenid, wobei unter den Pseudohalogeniden Cyanid, Rhodanid, Cyanat, Isocyanat, Thiocyanat und Isothiocyanat bevorzugt sind, und
L³ für einen Carbenliganden der allgemeinen Formel (III) oder (IV) gemäß Anspruch 2 steht und
L⁴ für einen neutralen Zweielektronen-Donor aus der Reihe Amine, Imine, Phosphane, Phosphite, Stibine, Arsine, CO, Carbonylverbindungen, Nitrile, Alkohole, Thiole, Ether und Thioether oder für einen neutralen Zweielektronen-Donor-Liganden aus der Gruppe der N-heterocyclischen Carbene der allgemeinen Formel A - E gemäß Anspruch 2 oder für einen Diaminocarben-Ligand der allgemeinen Formel (II) gemäß Anspruch 2 steht.

7. Verfahren zur Herstellung der Verbindungen gemäß Anspruch 1 bis 6 durch
a) Herstellung eines Carbenliganden der Formel (II) gemäß Anspruch 2 durch Deprotonierung eines Formamidiniumsalzes mit starken Basen oder nach der "Ammoniak-Methode" und
b) Umsetzung des Carbenliganden der Formel (II) mit geeigneten Übergangsmetallkomplexen in einer Ligandenaustauschreaktion.

8. Verfahren zur Herstellung der Verbindungen gemäß Anspruch 1 bis 6 durch Umsetzung von Verbindungen des Typs [R¹R²N-CH=NR³R⁴]⁺X⁻, wobei R¹ bis R⁴ die in den Ansprüchen 1 bis 6 genannte Bedeutung aufweisen, mit Übergangsmetallkomplexen in einer Salzmetathese.

9. Verfahren zur Herstellung der Verbindungen gemäß Anspruch 1 bis 6 durch Umsetzung von Verbindungen des Typs [R¹R²N-CH=NR³R⁴]⁺X⁻, wobei R¹ bis R⁴ die in den Ansprüchen 1 bis 6 genannte Bedeutung aufweisen, mit Alkalimetallalkoholaten und anschließender Umsetzung des entstehenden Alkoholataddukts mit Übergangsmetallkomplexen in einer Ligandenaustauschreaktion.

10. Verwendung der Verbindungen gemäß Anspruch 1 bis 6 als Katalysatoren.

11. Verwendung gemäß Anspruch 10 als Katalysatoren für die Hydrosilylierung, Hydroformylierung, Olefin-Metathese, Alkin-Metathese, Heck-Kupplung, Suzuki-Kupplung, Sonogashira-Kupplung, Cyclopropanierung oder Grignard-Kreuzkupplung.

12. Verwendung der Verbindungen gemäß Anspruch 5 als Katalysatoren in der Hydrosilylierung.

13. Verwendung der Verbindungen gemäß Anspruch 6 als Katalysatoren in der Olefin-Metathese.

## Claims

1. Compounds of the general structural formula (I), 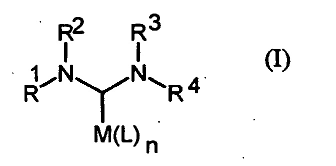 where
M is a transition metal of transition groups 6 to 8 of the Periodic Table,
the radicals R¹, R², R³ and R⁴ are identical or different and are hydrogen and/or hydrocarbon groups, where the hydrocarbon groups may be identical or different, straight-chain, branched or cyclic radicals selected from the group consisting of alkyl radicals having from 1 to 50 carbon atoms, alkenyl radicals having from 2 to 50 carbon atoms, alkynyl radicals having from 2 to 50 carbon atoms and aryl radicals having from 6 to 30 carbon atoms in which at least one hydrogen atom may be replaced by a functional group, and/or
R¹ and R² and/or R³ and R⁴ are part of a cyclic system which is identical or different, where the cyclic system comprises a carbon framework having from 2 to 20 carbon atoms and a nitrogen atom in formula (I), where the carbon atoms of R¹ and R² and/or R³ and R⁴ in formula (I) are not counted and where at least one hydrogen atom in the ring may be replaced by a functional group and/or at least one carbon atom of the ring may be replaced by a heteroatom selected from the group consisting of S, P, O and N, and/or
R¹ and/or R² and/or R³ and/or R⁴ are linked to one of the ligands L via a bridge having from 1 to 20 carbon atoms, where the carbon atoms of R¹, R² R³ and R⁴ are not counted, and
L are identical or independently different uncharged two-electron donors and/or are parts of a cyclic system and/or are anionic ligands, and
n is an integer.

2. Compounds according to Claim 1, wherein L are identical or independently different uncharged two-electron donors selected from the group consisting of amines, imines, phosphines, phosphites, stibines, arsines, CO, carbonyl compounds, nitriles, alcohols, thiols, ethers, thioethers and pyridines and/or
identical or independently different uncharged two-electron donors selected from the group consisting of N-heterocyclic carbenes of the general formula (A-E) 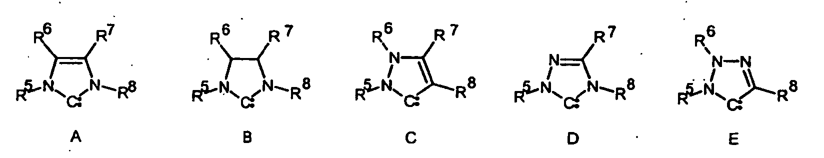 where
the radicals R⁵, R⁶, R⁷ and R⁸ are identical or different and are hydrogen and/or hydrocarbon groups, where the hydrocarbon groups are identical or different, straight-chain, branched or cyclic radicals selected from the group consisting of alkyl radicals having from 1 to 50 carbon atoms, alkenyl radicals having from 2 to 50 carbon atoms, alkynyl radicals having from 2 to 50 carbon atoms and aryl radicals having from 6 to 30 carbon atoms in which at least one hydrogen atom may be replaced by a functional group, and/or
the radicals R⁶ and R⁷ are identical or independently different halogen, nitro, nitroso, alkoxy, aryloxy, amido, carboxyl, carbonyl, thio or/and sulfonyl groups, and/or
identical or independently different uncharged two-electron donors selected from the group consisting of diaminocarbene ligands of the general formula (II), 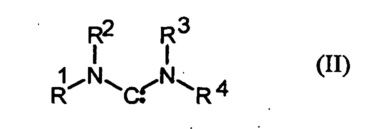 where
the radicals R¹, R², R³ and R⁴ are as defined in Claim 1, and/or
identical or independently different uncharged two-electron donor ligands selected from the group consisting of compounds of the general formulae (III) and (IV) 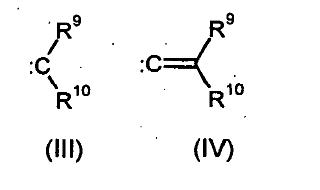 where
the radicals R⁹ and R¹⁰ are identical or different and are hydrogen or/and hydrocarbon groups, where the hydrocarbon groups are identical or different, straight-chain, branched or cyclic radicals selected from the group consisting of alkyl radicals having from 1 to 50 carbon atoms, alkenyl radicals having from 2 to 50 carbon atoms, alkynyl radicals having from 2 to 50 carbon atoms, aryl radicals having from 6 to 30 carbon atoms and silyl radicals in which at least one hydrogen atom may be replaced by an alkyl, aryl, alkenyl, alkynyl, metallocenyl, halogen, nitro, nitroso, hydroxy, alkoxy, aryloxy, amino, amido, carboxyl, carbonyl, thio or/and sulfonyl group.

3. Compounds according to Claim 1, wherein L are identical or independently different anionic ligands selected from the group consisting of halides, pseudohalides, hydroxides, alkoxides, thiolates, carboxylates and sulfonates, with preferred pseudohalides being cyanide, thiocyanate, cyanate, isocyanate and isothiocyanate.

4. Compounds according to Claim 1, wherein L is a cyclic diene, preferably a diene having from 5 to 10 carbon atoms.

5. Compounds according to Claim 1, wherein M is rhodium or iridium and n is 3 and L¹, L² and L³ are identical or/and independently different and/or are part of a cyclic system, where
L¹ is an anionic ligand selected from the group consisting of halide, alkoxide, carboxyl compound, thiolate and pseudohalide or is an uncharged two-electron donor selected from the group consisting of alkenes, alkynes, phosphines, amines, imines, phosphites, stibines, arsines, CO, carbonyl compounds, nitriles, alcohols, ethers, thiols and thioethers and
L² and L³ are identical or independently different and are uncharged two-electron donors selected from the group consisting of alkenes, alkynes, phosphines, amines, imines, phosphites, stibines, arsines, CO, carbonyl compounds, nitriles, alcohols, ethers, thiols and thiolethers or, in a preferred embodiment, are part of a cyclic diene; particular preference is given to L² and L³ together representing 1,5-cyclooctadiene.

6. Compounds according to Claim 1, wherein M is ruthenium or osmium and n is 4 and
L¹ and L² are identical or independently different and are each an anionic ligand selected from the group consisting of halide, alkoxide, carboxyl compound, thiolate and pseudohalide, with preferred pseudohalides being cyanide, thiocyanate, cyanate, isocyanate and isothiocyanate, and
L³ is a carbene ligand of the general formula (III) or (IV) according to Claim 2 and
L⁴ is an uncharged two-electron donor selected from the group consisting of amines, imines, phosphines, phosphites, stibines, arsines, CO, carbonyl compounds, nitriles, alcohols, thiols, ethers and thioethers or is an uncharged two-electron donor ligand selected from the group consisting of N-heterocyclic carbenes of the general formulae A - E according to claim 2 or is a diaminocarbene ligand of the general formula (II) according to Claim 2.

7. Process for preparing compounds according to any of Claims 1 to 6 by
a) preparing a carbene ligand of the formula (II) according to Claim 2 by deprotonation of a formamidinium salt with strong bases or by the "ammonium method" and
b) reacting the carbene ligand of the formula (II) with suitable transition metal complexes in a ligand exchange reaction.

8. Process for preparing compounds according to any of Claims 1 to 6 by reacting compounds of the type [R¹R²N-CH=NR³R⁴]⁺X⁻, where R¹ to R⁴ are as defined in Claims 1 to 6, with transition metal complexes in a salt metathesis.

9. Process for preparing compounds according to any of Claims 1 to 6 by reacting compounds of the type [R¹R²N-CH=NR³R⁴]⁺X⁻, where R¹ to R⁴ are as defined in Claims 1 to 6, with alkali metal alkoxides and subsequently reacting the alkoxide adduct formed with transition metal complexes in a ligand exchange reaction.

10. Use of the compounds according to any of Claims 1 to 6 as catalysts.

11. Use according to Claim 10 as catalysts for hydrosilylation, hydroformylation, olefin metathesis, alkyne metathesis, Heck coupling, Suzuki coupling, Sonogashira coupling, cyclopropanation or Grignard cross-coupling.

12. Use of the compounds according to Claim 5 as catalysts in hydrosilylation.

13. Use of the compounds according to Claim 6 as catalysts in olefin metathesis.

## Revendications

1. Composés répondant à la formule de structure générale (I) 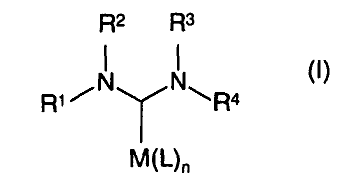 dans laquelle
M représente un métal de transition du 6^{ème} au 8^{ème} sous-groupe de la Classification Périodique,
les symboles R¹, R², R³ et R⁴, ayant des significations identiques ou différentes, représentent l'hydrogène et/ou des groupes hydrocarbonés, lesquels consistent en radicaux identiques ou différents, à chaîne droite, ramifiée ou cyclique choisis parmi les radicaux alkyle en C₁-C₅₀, les radicaux alcényle en C₂-C₅₀, les radicaux alcynyle en C₂-C₅₀ et les radicaux aryle en C₆-C₃₀, dans lesquels le cas échéant au moins un atome d'hydrogène est remplacé par un groupe fonctionnel, et/ou
R¹ et R² et/ou R³ et R⁴ sont des parties de systèmes cycliques identiques ou différents, consistant eux-mêmes en un squelette carboné de 2 à 20 atomes de carbone et un des atomes d'azote de la formule (I), les atomes de carbone de R¹ et R² et/ou R³ et R⁴ de la formule (I) n'étant pas comptés dans ce nombre, et le cas échéant au moins un atome d'hydrogène du cycle est remplacé par un groupe fonctionnel et/ou le cas échéant au moins un atome de carbone du cycle est remplacé par un hétéroatome choisi parmi S, P, O et N, et/ou
R¹ et/ou R² et/ou R³ et/ou R⁴ sont reliés à l'un des ligands L par un pont de 1 à 20 atomes de carbone, les atomes de carbone de R¹, R², R³ et R⁴ n'étant pas comptés dans ce nombre, et
les symboles L représentent des donateurs neutres de deux électrons, identiques ou différents et indépendants les uns des autres et/ou des parties de systèmes cycliques et/ou des ligands anioniques, et
n est un nombre entier.

2. Composés selon la revendication 1, pour lesquels les symboles L représentent des donateurs neutres de deux électrons, identiques ou différents et indépendants les uns des autres, choisis parmi les amines, les imines, les phosphanes, les phosphites, les stibines, les arsines, CO, les dérivés carbonylés, les nitriles, les alcools, les thiols, les éthers, les thioéthers et les pyridines et/ou
des donateurs neutres de deux électrons, identiques ou différents, et indépendants les uns des autres, du groupe des carbènes hétérocycliques azotés de formules générales A à E 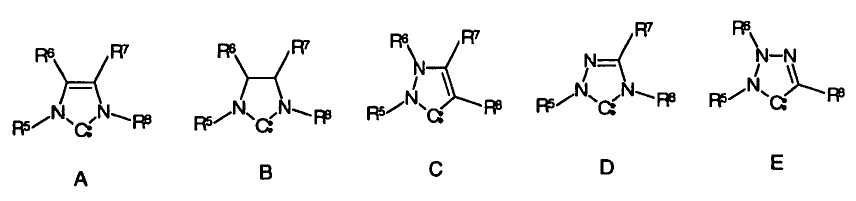 dans lesquelles
les symboles R⁵, R⁶, R⁷ et R⁸, ayant des significations identiques ou différentes, représentent l'hydrogène et/ou des groupes hydrocarbonés, lesquels consistent en radicaux identiques ou différents, à chaîne droite, ramifiée ou cyclique, du groupe des radicaux alkyle en C₁-C₅₀, des radicaux alcényle en C₂-C₅₀, des radicaux alcynyle en C₂-C₅₀ et des radicaux aryle en C₆-C₃₀, dans lesquels le cas échéant au moins un atome d'hydrogène est remplacé par un groupe fonctionnel et/ou
les symboles R⁶ et R⁷, ayant des significations identiques ou différentes et indépendantes l'une de l'autre, représentent chacun un groupe halogéno, nitro, nitroso, alcoxy, aryloxy, amido, carboxyle, carbonyle, thio et/ou sulfonyle, et/ou
ayant des significations identiques ou différentes et indépendantes l'une de l'autre, ils représentent des donateurs neutres de deux électrons du groupe des ligands du type diaminocarbène de formule générale (II) 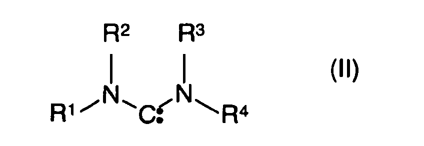 dans laquelle
les symboles R¹, R², R³ et R⁴ ont les significations indiquées dans la revendication 1, et/ou
ayant des significations identiques ou différentes et indépendantes l'une de l'autre, ils représentent des ligands-donateurs de deux électrons neutres du groupe des composés de formules générales (III) et (IV) 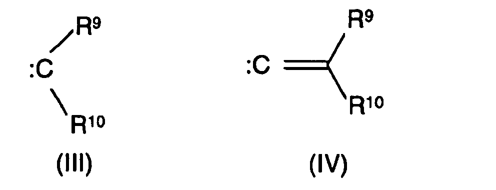 dans lesquelles
les symboles R⁹ et R¹⁰, ayant des significations identiques ou différentes, représentent l'hydrogène et/ou des groupes hydrocarbonés, lesquels consistent en radicaux identiques ou différents, à chaîne droite, ramifiée ou cyclique, choisis parmi les radicaux alkyle en C₁-C₅₀, les radicaux alcényle en C₂-C₅₀, les radicaux alcynyle en C₂-C₅₀, les radicaux aryle en C₆-C₃₀ et les radicaux silyle, dans lesquels le cas échéant au moins un atome d'hydrogène est remplacé par un groupe alkyle, aryle, alcényle, alcynyle, métallocényle, halogéno, nitro, nitroso, hydroxy, alcoxy, aryloxy, amino, amido, carboxyle, carbonyle, thio et/ou sulfonyle.

3. Composés selon la revendication 1, pour lesquels les symboles L ayant des significations identiques ou différentes et indépendantes les unes des autres, représentent des ligands anioniques choisis parmi les halogénures, les pseudo-halogénures, les hydroxydes, les alcoolates, les thiolates, les carboxylates et les sulfonates, les pseudo-halogénures consistant de préférence en cyanures, thiocyanures, cyanates, isocyanates, thiocyanates et isothiocyanates.

4. Composés selon la revendication 1, pour lesquels L représente un diène cyclique, de préférence un diène en C₅-C₁₀.

5. Composés selon la revendication 1, pour lesquels M représente le rhodium ou l'iridium et n est égal à 3, L¹, L² et L³ ayant des significations identiques et/ou différentes et indépendantes les unes des autres et/ou formant une partie d'un système cyclique,
L¹ représentant un ligand anionique choisi parmi les halogénures, les alcoolates, les dérivés carboxylés, les thiolates et les pseudo-halogénures ou un donateur neutre de deux électrons choisi parmi les alcènes, les alcynes, les phosphanes, les amines, les imines, les phosphites, les stibines, les arsines, CO, les dérivés carbonylés, les nitriles, les alcools, les éthers, les thiols et les thioéthers et
L² et L³, ayant des significations identiques ou différentes et indépendantes l'une de l'autre, représentent des donateurs neutres de deux électrons choisis parmi les alcènes, les alcynes, les phosphanes, les amines, les imines, les phosphites, les stibines, les arsines, CO, les dérivés carbonylés, les nitriles, les alcools, les éthers, les thiols et les thioéthers ou bien de préférence une partie d'un diène cyclique, et dans les meilleures conditions, L² et L³ représentent ensemble le 1,5-cyclooctadiène.

6. Composés selon la revendication 1, pour lesquels M représente le ruthénium ou l'osmium et n est égal à 4, et
L¹ et L², ayant des significations identiques ou différentes et indépendantes l'une de l'autre, représentent chacun un ligand anionique choisi parmi les halogénures, les alcoolates, les dérivés carboxylés, les thiolates et les pseudo-halogénures, les pseudo-halogénures préférés consistant en les cyanures, les thiocyanures, les cyanates, les isocyanates, les thiocyanates et les isothiocyanates, et
L³ représente un ligand du type carbène de formule générale (III) ou (IV) de la revendication 2 et
L⁴ représente un donateur neutre de deux électrons choisi parmi les amines, les imines, les phosphanes, les phosphites, les stibines, les arsines, CO, les dérivés carbonylés, les nitriles, les alcools, les thiols, les éthers et les thioéthers ou un ligand-donateur neutre de deux électrons choisi parmi les carbènes hétérocycliques azotés de formules générales A à E de la revendication 2 ou bien un ligand du type diaminocarbène de formule générale (II) de la revendication 2.

7. Procédé pour la préparation des composés selon les revendications 1 à 6, consistant à
a) préparer un ligand du type carbène de formule (II) de la revendication 2 par déprotonation d'un sel de formamidinium à l'aide d'une base forte ou par le "procédé à l'ammoniac" et
b) faire réagir le ligand de type carbène de formule (II) avec des complexes appropriés de métaux de transition dans une réaction d'échange de ligands.

8. Procédé pour la préparation des composés selon les revendications 1 à 6, par réaction de composés du type [R¹R²N-CH=NR³R⁴]⁺X⁻, pour lesquels R¹ à R⁴ ont les significations indiquées dans les revendications 1 à 6, avec des complexes de métaux de transition dans une réaction de double décomposition entre sels.

9. Procédé pour la préparation des composés selon les revendications 1 à 6 par réaction de composés du type [R¹R²N-CH=NR³R⁴]⁺X⁻, pour lesquels R¹ à R⁴ ont les significations indiquées dans les revendications 1 à 6, avec des alcoolates de métaux alcalins, suivie d'une réaction entre l'adduct d'alcoolate obtenu et des complexes de métaux de transition dans une réaction d'échange de ligands.

10. Utilisation des composés selon les revendications 1 à 6 en tant que catalyseurs.

11. Utilisation selon la revendication 10 en tant que catalyseurs pour l'hydrosilylation, l'hydroformylation, la métathèse d'oléfines, la métathèse d'alcynes, la condensation de Heck, la condensation de Suzuki, la condensation de Sonogashira, la cyclopropanation ou la condensation croisée de Grignard.

12. Utilisation des composés selon la revendication 5 en tant que catalyseurs pour l'hydrosilylation.

13. Utilisation des composés selon la revendication 6 en tant que catalyseurs pour la métathèse d'oléfines.
